(19) Europäisches Patentamt
European Patent Office
Office européen des brevets

(11) Publication number: **0 420 120 B1**

(12) **EUROPEAN PATENT SPECIFICATION**

(45) Date of publication of patent specification: **31.08.94**

(51) Int. Cl.$^5$: **C07C 215/60**, A61K 31/135

(21) Application number: **90118338.4**

(22) Date of filing: **25.09.90**

(54) **Optically active benzyl alcohol compound and pharmaceutical composition.**

(30) Priority: **28.09.89 JP 250424/89**
**01.08.90 JP 202476/90**

(43) Date of publication of application:
**03.04.91 Bulletin 91/14**

(45) Publication of the grant of the patent:
**31.08.94 Bulletin 94/35**

(84) Designated Contracting States:
**CH DE FR GB IT LI SE**

(56) References cited:

**ARZNEIMITTEL FORSCHUNG, August 1980, pages 1272-1278; S. KUBO et al.: "Pharmacological studies of 1-(2-chloro-4-hydroxyphenyl)2-t-butylaminoethanol (HOKU-81), a new bronchodilator"**

(73) Proprietor: **HOKURIKU PHARMACEUTICAL CO., LTD.**
**1-Chome, 3-14, Tatekawacho,**
**Katsuyama-shi**
**Fukui-ken 911 (JP)**

(72) Inventor: **Ito, Yasuo**
**11-14, Moto-machi 3-chome**
**Katsuyama-shi, Fukui-ken (JP)**
Inventor: **Kato, Hideo**
**5-8, Kentoku 3-chome**
**Fukui-shi, Fukui-ken (JP)**
Inventor: **Koshinaka, Eiichi**
**6-3, Asahi-cho 2-chome**
**Katsuyama-shi, Fukui-ken (JP)**
Inventor: **Kurata, Sakae**
**6-56, Asahi-cho 1-chome**
**Katsuyama-shi, Fukui-ken (JP)**
Inventor: **Morikawa, Koji**
**6-63, Asahi-cho 2-chome**
**Katsuyama-shi, Fukui-ken (JP)**

(74) Representative: **Werner, Hans-Karsten, Dr. et al**
**Patentanwälte**
**Von Kreisler-Selting-Werner**
**Postfach 10 22 41**
**D-50462 Köln (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to an optically active benzyl alcohol compound and a pharmacologically acceptable salt thereof. More specifically, the present invention relates to a levo-rotatory enantiomer of benzyl alcohol compound represented by the following formula (I):

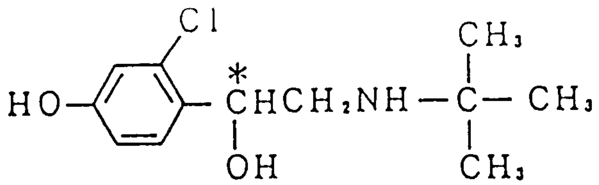

(I)

wherein * C represents an asymmetric carbon atom; and pharmacologically acceptable salt of the compound, which excellently activate β -adrenergic receptors and is useful for pharmaceuticals, i.e., a uterine smooth muscle relaxing agent or a bladder smooth muscle relaxing agent.

The present invention also relates to the method for preparing the levo-rotatory enantiomer and pharmaceutical compositions comprising an effective amount of the levo-rotatory enantiomer.

Description of the Prior Art

Japanese Patent Publication No. 6625/1980 discloses racemic benzyl alcohol compound represented by the following formula (II):

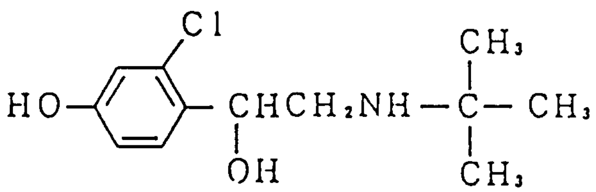

( II )

which was first synthesized by the inventors of the present invention and was expected to be developed as an agent for the treatment of respiratory obstruction such as bronchial asthma since the racemic compound has bronchodilating activity caused by activation of $\beta_2$-adrenergic receptors.

Since the compound represented by the above formula (II) has an asymmetric carbon atom in the benzyl position, one skilled in the art may understand that the racemic compound consists of two enantiomers, however, the above-mentioned patent document neither discloses nor teaches the enantiomers per se nor the pharmacological activity of the enantiomer, i.e., uterine smooth muscle-relaxing activity and bladder smooth muscle relaxing activity of one of the enantiomers.

The treatment and control of premature labor is quite important since premature labor sometimes results in premature delivery or perinatal death. The etiology of the premature labor has not been successfully determined, however, agents for relaxing uterine smooth muscle are expected to be useful for the treatment of premature labor since it is believed to be caused mainly by premature oxytocia.

For example, progesterone and/or progestogens, Isoxsuprine (The Merck Index, 11th edition, 5128), or Ritodrine (The Merck Index, 11th edition, 8228) have been clinically used, however, they are considered to be insufficient in curative effect and cause undesirable adverse reactions when administered to patients.

The number of patients suffering from urinary incontinence or night enuresis, has recently increased. Agents for relaxing urinary bladder smooth muscle are expected to be useful for the treatment of these diseases since every patient suffering from these diseases has a bladder with an increased intravesical pressure, i.e., a contracted bladder.

SUMMARY OF THE INVENTION

An object of the present invention is to provide a compound having uterine smooth muscle relaxing activity and bladder smooth muscle relaxing activity, and to provide a uterine smooth muscle relaxing agent and a bladder smooth muscle relaxing agent comprising said compound, which eliminates the undesirable

adverse reactions of prior art compounds.

Another object of the present invention is to provide a method for preparing said compound.

A further object of the present invention is to provide a pharmaceutical composition for the treatment of such diseases as premature labor or urinary incontinence comprising said compound together with a pharmaceutically acceptable carrier or coating.

The inventors of the present invention have conducted various studies to achieve the foregoing objects. First, they succeeded optically resolving the benzyl alcohol compound represented by the above formula (II) to obtain a levo-rotatory enantiomer and dextro-rotatory enantiomer of said compound. They then found that the objects of the present invention could be effectively achieved by providing the levo-rotatory enantiomer of the compound of formula (II). This compound has excellent relaxing activities on uterine smooth muscle and bladder smooth muscle, and thus is useful for the treatment of premature labor, urinary incontinence, or night enuresis without causing undesiable adverse reactions such as positive chronotropic effect.

In accordance with an embodiment of the present invention, the present invention provides a levo-rotatory enantiomer of a benzyl alcohol compound represented by the following formula (I):

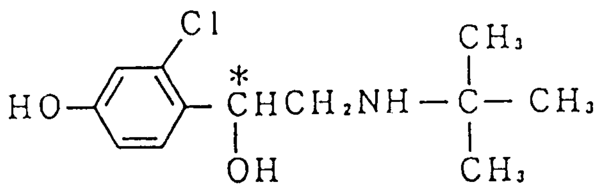

(I)

wherein * C represents an asymmetric carbon atom. The invention also provides a pharmacologically acceptable salt of the compound, and a method of preparing the compound.

In accordance with further embodiments of the present invention, there are provided a uterine smooth muscle relaying agent and a bladder smooth muscle relaxing agent comprising an effective amount of a benzyl alcohol compound represented by the formula (I), and a pharmaceutical composition for the treatment of premature labor or urinary incontinence comprising an effective amount of a compound represented by the formula (I).

Further objects, features and advantages of the present invention will become apparent from the Description of the Preferred Embodiments which follows, when read in light of the attached Examples.

## DESCRIPTION OF THE PREFERRED EMBODIMENTS

The pharmaceutically acceptable salts of the optically active benzyl alcohol compound of the present invention represented by the above formula (I) may be acid addition salts or alkali addition salts. Examples of the acid addition salts include mineral acid salts such as, for example, hydrochloride, hydrobromide, hydroiodide, nitrate, sulfate, or phosphate, and organic acid salts such as, for example, acetate, maleate, fumarate, citrate, oxalate, malate, methanesulfonate, p-toluenesulfonate, mandelate, 10-camphorsulfonate, tartarate, or succinate. Examples of the alkali addition salts include sodium, potassium, and calcium salts.

The optically active benzyl alcohol compound of the present invention represented by the above formula (I) may be prepared by a method according to the present invention comprising the steps of converting the racemic benzyl alcohol compound represented by the above formula (II) to the salt thereof by using a reagent for optical resolution, fractionally recrystallizing the resulting salt, and followed by converting the salt to a free base, if necessary.

Examples of the reagent for the optical resolution used in the method according to the present invention include D-camphor-10-sulfonic acid, L-camphor-10-sulfonic acid, (+)-dibenzoyl-D-tartaric acid, (-)-dibenzoyl-L-tartaric acid, L-(+)-tartaric acid, D-(-)-tartaric acid, L-(+)-mandelic acid, D-(-)-mandelic acid, *d*-camphoric acid, D-malic acid, L-malic acid, (+)-di-p-toluoyl-D-tartaric acid, (-)-di-toluoyl-L-tartaric acid, (-)-menthyloxyacetic acid, (-)-diacetyl-L-tartaric acid, (+)-monomethyl-D-tartaric acid, (-)-monomethyl-L-tartaric acid, (-)-diacetone-2-ketogulonic acid, (-)-quinic acid, D-glutamic acid, L-glutamic acid, (S)-(-)-pyrrolidone-5-carboxylic acid, (R)-(-)-2-phenylpropionic acid, (S)-(+)-2-phenylpropionic acid, (S)-1-(2-naphthylsulfonyl)-pyrrolidine-2-carboxylic acid, and (S)-1-(4-toluenesulfonyl)pyrrolidine-2-carboxylic acid.

Examples of the solvent used in the fractional recrystallization include, for example, water; alcohols such as for example methanol, ethanol, or isopropanol; halogenated hydrocarbons such as for example chloroform, dichloromethane, dichloroethane, or carbon tetrachloride; ketones such as for example acetone

or methyl ethyl ketone; ethers such as for example diethyl ether, di-isopropyl ether, or dioxane; aromatic hydrocarbons such as for example benzene, toluene, or xylene; saturated hydrocarbons such as for example hexane, pentane, or cyclohexane; nitriles such as for example acetonitrile; esters such as for example ethyl acetate or ethyl formate; amides such as for example N,N-dimethylformamide or N,N-dimethylacetamide; other organic solvents such as for example dimethyl sulfoxide or nitromethane; and mixtures of the above solvents. The fractional recrystallization may be carried out at room temperature or at an elevated temperature.

The levo-rotatory enantiomer of the present invention represented by the above formula (I) has relaxing activities on uterine smooth muscle and bladder smooth muscle. Thus, a uterine smooth muscle relaxing agent and a bladder smooth muscle relaxing agent comprising the levo-rotatory enantiomer is useful for the treatment of premature labor or urinary incontinence. The levo-rotatory enantiomer of the present invention may preferably be administered orally or parenterally to a patient as a pharmaceutical composition comprising an effective amount of the levo-rotatory enantiomer of the present invention together with a pharmaceutically acceptable carrier or coating. The pharmaceutical composition of the present invention may be in the form of, for example, compositions for oral administration such as for example tablets, capsules, powders, granules, subtilized granules, syrups, or dry syrups; or in the form of injection or suppository. The composition may be manufactured by a known method by using pharmaceutically acceptable carriers or coatings widely used for pharmaceutical compositions. For the preparation of the pharmaceutical composition suitable for oral administration or suppository, the carrier or coating may comprise an excipient such as for example glucose, lactose, D-mannitol, starch, hydroxypropylstar ch, or crystalline cellulose; a disintegrant such as for example carboxymethylcellulose, carboxypropylcellulose, or calcium carboxymethylcellulose; a binder such as for example hydroxypropylcellulose, hydroxypropylmethylcellulose, or polyvinylpyrrolidone; a lubricant such as for example magnesium stearate or talc; a coating agent such as for example hydroxypropylmethylcellulose or sucrose; or a base such as for example polyethyleneglycol or hard fat. The pharmaceutical composition suitable for injection or precursor for such composition may comprise the following: a solubilizing agent or a solubilizer for making an aqueous solution, e.g., distilled water for injection, saline, or propylene glycol; a pH adjusting agent such as for example an inorganic or organic acid or inorganic base; or a stabilizer or an isotonicity agent such as for example sodium chloride or glucose.

The dose of the pharmaceutical composition of the present invention for an adult patient may generally be from about 0.01 to 10 mg per day for oral or parenteral administration, which may be increased or decreased depending on the conditions of the patient to be treated.

The present invention will be further illustrated by the following Examples which are given by way of illustration only and are not to be construed as limiting.

## Examples

The following example shows the excellent effectiveness of the compound of the present invention.

### 1. Effects on isolated uterine smooth muscle of rats

Non-pregnant female Wistar rats weighing about 200 g were killed and the uteruses were isolated. Each uterine preparation (15 mm in length) was mounted vertically in an organ bath containing modified Locke-Ringer solution of following composition: 150 mM NaCl, 5.4 mM KCl, 0.36 mM $CaCl_2$, 0.19 mM $MgCl_2$, 4.8 mM $NaHCO_3$, 0.15 mM $KH_2PO_4$, 0.56 mM $Na_2HPO_4$ and 2.8 mM Glucose. The bath medium was maintained at 37 °C and was equilibrated with a gas mixture consisting of 95% $O_2$ + 5% $CO_2$. The uterine preparations were pre-contracted with oxytocin ($10^{-2}$ U/*ml*) and the muscle relaxations induced by the test compounds were recorded isotonically. The test compounds were added cumulatively to the bath. $ED_{50}$ values for concentration-response curves which produce 50% relaxations of the maximal relaxations ($10^{-6}$ M isoproterenol) were calculated. Results are shown in Table 1.

Table 1

| Test compoumds | Relaxation $ED_{50}$ (M) |
|---|---|
| (-)-enantiomer [a] | $5.6 \times 10^{-9}$ |
| (+)-enantiomer [b] | $7.4 \times 10^{-7}$ |
| ritodrine [c] | $1.4 \times 10^{-7}$ |

a): the compound of the present invention
b): (+)-enantiomer of the compound of formula (II)
c): positive control

As shown in Table 1, the effect compound of the present invention is about 130 fold greater than the (+)-enantiomer and 25 fold greater than ritodrine, respectively, thus those skilled in the art can readily understand that the compound of the present invention is very useful as an agent for relaxing uterine smooth muscle.

2. Effects on isolated bladder smooth muscle of rabbits

Male albino rabbits weighing about 2.7 kg were killed and the bladders were isolated. Each bladder preparation (3 mm in width and 10 mm in length) was mounted vertically in an organ bath containing Krebs-Henseleit solution of following composition: 118 mM NaCl, 4.7 mM KCl, 2.55 mM $CaCl_2$, 1.18 mM $MgSO_4$, 1.18 mM $KH_2PO_4$, 24.88 mM $NaHCO_3$, and 11.1 mM Glucose. The bath medium was maintained at 37°C and was equilibrated with a gas mixture consisting of 95% $O_2$ + 5% $CO_2$. A resting tension of 0.5 g was applied and the response was recorded isometrically. The test compounds were added cumulatively to the bath. $ED_{50}$ values for concentration-response curves which produce 50% relaxations of the maximal relaxations ($10^{-6}$ M isoproterenol) were calculated. The results are shown in Table 2.

Table 2

| Test compoumd | Relaxation $ED_{50}$ (M) |
|---|---|
| (-)-enantiomer [a] | $2.6 \times 10^{-10}$ |

a): the inventive compound

As shown in Table 2, the inventive compound has a potent relaxing effect on the bladder smooth muscle and is very useful as an agent for relaxing bladder smooth muscle, while (+)-enantiomer does not have little relaxing effect on the bladder smooth muscle.

3. Effects on isolated atrium of guinea-pigs

Male Hartley guinea-pigs weighing about 500 g were killed and the atria were isolated. Each right atrium was mounted vertically in an organ bath containing Krebs-Henseleit solution and the spontaneous beating rate was recorded. The bath medium was maintained at 30°C and was equilibrated with a gas mixture consisting of 95% $O_2$ + 5% $CO_2$. The test compounds were added cumulatively to the bath. $ED_{50}$ values for concentration-response curves which produce 50% increases of the basal spontaneous beating rate were calculated. The results are shown in Table 3. The selcctive relaxing activities of the compound of the present invention on the uterine and the bladder smooth muscle were compared with positive chronotropic effect in the atrium. The results are also shown in Table 3.

Table 3

| Test compoumd | Positive chronotropic effect $ED_{50}$ (M) | Selectivity | |
|---|---|---|---|
| | | Uterus[b)] | Bladder[c)] |
| (-)-enantiomer [a)] | $1.2 \times 10^{-7}$ | 21 | 4 6 2 |

a): the inventive compound
b): $ED_{50}$ value (positive chronotropic effect in the atrium) / $ED_{50}$ value (relaxing effect on the uterine smooth muscle)
c): $ED_{50}$ value (positive chronotropic effect in the atrium) / $ED_{50}$ value (relaxing effect on the bladder smooth muscle)

As shown in Table 3, the compound of the present invention has potent relaxing activities on uterine and bladder smooth muscle and while it has little effect in the atrium, thus the compound of the present invention is very useful to eliminate adverse reactions such as positive chronotropic action when administered as an agent for reluxing uterine and bladder smooth muscle.

Example 1

(-)-α-[(tert-Butylamino)methyl]-2-chloro-4-hydroxybenzyl alcohol

(1) (±)-α-[(tert-Butylamino)methyl]-2-chloro-4-hydroxybenzyl alcohol (97.49 g) and 100.12 g of D-10-camphorsulfonic acid were dissolved in 3000 ml of methanol and the solution was concentrated. Ethyl acetate (500 ml) was added to the residue and the precipitate was collected to give 138.71 g of colorless crystals, which were recrystallized from acetone-ethyl acetate (3:4). The optical rotation of the obtained precipitate and the residual crystals given from the filtrate by concentration was measured. The above-described recrystallization and measurement of the optical rotation of the precipitate or residual crystals from the filtrate were repeated until levo-rotatory crystals were obtained. The obtained levo-rotatory crystals were collected and then recrystallized twice from acetone-ethyl acetate (3:4) to give 11.79 g of (-)-α-[(tert-butylamino)methyl]-2-chloro-4-hydroxybenzyl alcohol • D-10-camphorsulfonic acid salt as colorless needles, mp 193-195 °C .

| Analysis for $C_{12}H_{18}ClNO_2 \cdot C_{10}H_{16}O_4S$: | | | |
|---|---|---|---|
| Calculated | C, 55.51; | H, 7.20; | N, 2.94 |
| Found | C, 55.35; | H, 7.62; | N, 2.67 |

Specific rotation $[\alpha]_D^{20}$ -23.0° (c = 1, MeOH)

(2) (-)-α-[(tert-Butylamino)methyl]-2-chloro-4-hydroxybenzyl alcohol • D-10-camphorsulfonic acid salt (11.79 g) was dissolved in 150 ml of water. The solution was made alkaline with potassium carbonate and then extracted with ethyl acetate. The ethyl acetate layer was dried and concentrated. The residue was recrystallized from ethyl acetate to give 2.93 g of the desired compound as colorless prisms, mp 158-159 °C .

NMR spectrum δ ($CD_3OD$) ppm: 1.16(9H, s), 2.61(1H, dd, J = 11.5, 9Hz), 2.81(1H, dd, J = 11.5, 3.5Hz), 5.07(1H, dd, J = 9, 3.5Hz), 6.73(1H, dd, J = 9.5, 2.5Hz), 6.75(1H, d, J = 2.5Hz), 7.37(1H, d, J = 9.5Hz)

| Analysis for $C_{12}H_{18}ClNO_2$ | | | |
|---|---|---|---|
| Calculated | C, 59.14; | H, 7.44; | N, 5.75 |
| Found | C, 58.89; | H, 7.92; | N, 5.36 |

Specific rotation $[\alpha]_D^{22}$ -52.7° (c = 1, MeOH)

Example 2

(-)-α-[(tert-Butylamino)methyl]-2-chloro-4-hydroxybenzyl alcohol

(1) (±)-α-[(tert-Butylamino)methyl]-2-chloro-4-hydroxybenzyl alcohol (10.0 g) and 6.16 g of L-tartaric acid were dissolved in 130 ml of methanol and the solution was concentrated. Acetone (50 ml) was added to the residue and the precipitate was collected to give 15.50 g of colorless crystals. The crystals were recrystallized from a mixture of 50 ml of methanol and 100 ml of acetone and the precipitates were filtered off. The filtrate was concentrated to give 13.23 g of colorless crystals. The crystals were recrystallized twice from methanol-acetone (1:2) and the second filtrate was concentrated to give 8.00 g of colorless crystals. The crystals were recrystallized from methanol-acetone (1:2) and the filtrate was concentrated to give colorless crystals, which were recrystallized twice from methanol-acetone (1:2) to give 0.52 g of colorless crystals. The crystals obtained were recrystallized from isopropanol to give (-)-α-[(tert-butylamino)methyl]-2-chloro-4-hydroxybenzyl alcohol • L-tartaric acid salt as colorless needles, mp 185-187 °C .

| Analysis for $C_{12}H_{18}ClNO_2$ • $C_4H_6O_6$ | | | |
|---|---|---|---|
| Calculated | C, 48.80; | H, 6.14; | N, 3.56 |
| Found | C, 48.45; | H, 6.27; | N, 3.56 |

Specific rotation $[\alpha]_D^{20}$ -35.5° (c = 0.1, MeOH)

(2) (-)-α-[(tert-Butylamino)methyl]-2-chloro-4-hydroxybenzyl alcohol • L-tartaric acid salt (0.42 g) was dissolved in 4.5 ml of water. The solution was made alkaline with potassium carbonate and then extracted with ethyl acetate. The ethyl acetate layer was dried and concentrated. The residue was recrystallized from ethyl acetate to give 0.17 g of the desired compound as colorless prisms.

The physical properties of the compound obtained was consistent with those obtained in Example 1.

The desired compound thus prepared was converted to the following salts in the usual manner.

Maleic acid salt

Colorless needles, mp 173-175 °C (acetone)

| Analysis for $C_{12}H_{18}ClNO_2$ • $C_4H_4O_4$ | | | |
|---|---|---|---|
| Calculated | C, 53.41; | H, 6.16; | N, 3.89 |
| Found | C, 53.49; | H, 6.21; | N, 3.80 |

Specific rotation $[\alpha]_D^{20}$ -57.6° (c = 0.5, MeOH)

Fumaric acid salt

Colorless prisms, mp 213-215 °C ($H_2O$-acetone)

| Analysis for $C_{12}H_{18}ClNO_2$ • $1/2C_4H_4O_4$ | | | |
|---|---|---|---|
| Calculated | C, 55.72; | H, 6.68; | N, 4.64 |
| Found | C, 55.62; | H, 6.61; | N, 4.64 |

Specific rotation $[\alpha]_D^{20}$ -56.1° (c = 0.1, MeOH)

D-Tartaric acid salt

Colorless prisms, mp 197-199 °C (iso-PrOH)

| Analysis for $C_{12}H_{18}ClNO_2 \cdot C_4H_6O_6$ | | | |
|---|---|---|---|
| Calculated | C, 48.80; | H, 6.14; | N, 3.56 |
| Found | C, 48.83; | H, 6.13; | N, 3.69 |

Specific rotation $[\alpha]_D^{20}$ -51.5° (c = 0.1, MeOH)

L-Tartaric acid salt

Colorless needles, mp 185-187 °C (iso-PrOH)

| Analysis for $C_{12}H_{18}ClNO_2 \cdot C_4H_6O_6$ | | | |
|---|---|---|---|
| Calculated | C, 48.80; | H, 6.14; | N, 3.56 |
| Found | C, 48.45; | H, 6.27; | N, 3.56 |

Specific rotation $[\alpha]_D^{20}$ -35.5° (c = 0.1, MeOH)

Succinic acid salt

White crystals, mp 189-191 °C (acetone)

| Analysis for $C_{12}H_{18}ClNO_2 \cdot 1/2C_4H_6O_4$ | | | |
|---|---|---|---|
| Calculated | C, 55.54; | H, 6.99; | N, 4.63 |
| Found | C, 55.44; | H, 7.01; | N, 4.87 |

Specific rotation $[\alpha]_D^{20}$ -62.0° (c = 0.1, MeOH)

Sulfuric acid salt

Colorless prisms, mp 216-218 °C ($H_2O$)

| Analysis for $C_{12}H_{18}ClNO_2 \cdot 1/2H_2SO_4$ | | | |
|---|---|---|---|
| Calculated | C, 49.23; | H, 6.54; | N, 4.78 |
| Found | C, 48.99; | H, 6.45; | N, 4.84 |

Specific rotation $[\alpha]_D^{20}$ -64.5° (c = 0.1, MeOH)

p-Toluenesulfonic acid salt

Colorless needles, mp 174-175 °C (acetone)

| Analysis for $C_{12}H_{18}ClNO_2 \cdot C_7H_8O_3S$ | | | |
|---|---|---|---|
| Calculated | C, 54.87; | H, 6.30; | N, 3.37 |
| Found | C, 54.65; | H, 6.13; | N, 3.27 |

Specific rotation $[\alpha]_D^{20}$ -49.2° (c = 0.1, MeOH)

Example 3

Capsules of a pharmaceutical composition according to the present invention are prepared in the usual manner using the following constituents:

| | |
|---|---|
| Compound of the present invention | 0.25 mg |
| Lactose | q.s. |
| Corn starch | 45 mg |
| Magnesium stearate | 1 mg |
| | 130 mg |

Example 4

Tablets of a pharmaceutical composition according to the present invention are prepared in the usual manner using the following constituents:

| | |
|---|---|
| Compound of the present invention | 0.25 mg |
| Lactose | q.s. |
| Corn starch | 30 mg |
| Carboxymethylcellulose calcium | 10 mg |
| Polyvinylpyrrolidone | 3 mg |
| Magnesium stearate | 1 mg |
| | 130 mg |

Example 5

Granules of a pharmaceutical composition according to the present invention are prepared in the usual manner using the following constituents:

| | |
|---|---|
| Compound of the present invention | 0.5 mg |
| Lactose | q.s. |
| Hydroxypropylstarch | 200 mg |
| Hydroxypropylcellulose | 20 mg |
| Talc | 5 mg |
| | 1000 mg |

Example 6

Dry syrups of a pharmaceutical composition according to the present invention are prepared in the usual manner using the following constituents:

| | |
|---|---|
| Compound of the present invention | 0.5 mg |
| Sucrose | q.s. |
| | 1000 mg |

Example 7

Injections of a pharmaceutical composition according to the present invention are prepared in the usual manner using the following constituents:

| | |
|---|---|
| Compound of the present invention<br>Sodium chloride<br>Distilled water for injection | 2.5 mg<br>40 mg<br>q.s. |
| | 5 ml |

Example 8

Suppositories of a pharmaceutical composition according to the present invention are prepared in the usual manner using the following constituents:

| | |
|---|---|
| Compound of the present invention<br>Hard fat | 0.25 mg<br>1299.75 mg |
| | 1300 mg |

**Claims**

**1.** A levo-rotatory enantiomer of benzyl alcohol compound represented by the following formula (I):

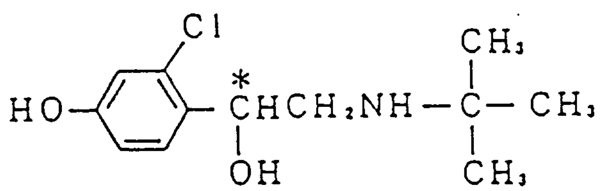

(I)

wherein * C represents an asymmetric carbon atom; and pharmacologically acceptable salts of the compound.

**2.** A method for preparing a levo-rotatory enantiomer of benzyl alcohol compound represented by the following formula (I):

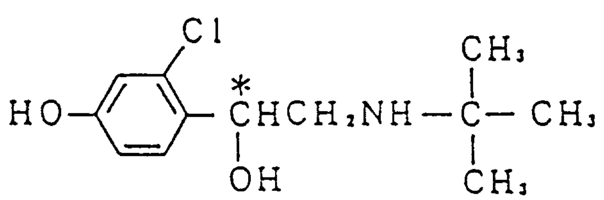

(I)

wherein * C represents an asymmetric carbon atom, and pharmacologically acceptable salt of the compound, comprising the steps of converting a racemic benzyl alcohol compound represented by the following formula (II):

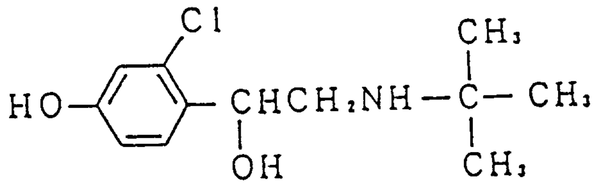

(II)

to a salt thereof by using a reagent for optical resolution, fractionally recrystallizing the salt, and, if necessary, converting the salt to a free base.

**3.** The method for preparing the levo-rotatory enantiomer according to claim 2, wherein the reagent for optical resolution is selected from the group consisting of D-camphor-10-sulfonic acid, L-camphor-10-sulfonic acid, (+)-dibenzoyl-D-tartaric acid, (-)-dibenzoyl-L-tartaric acid, L-(+)-tartaric acid, D-(-)-tartaric acid, L-(+)-mandelic acid, D-(-)-mandelic acid, *d*- camphoric acid, D-malic acid, L-malic acid, (+)-di-p-toluoyl-D-tartaric acid, (-)-di-toluoyl-L-tartaric acid, (-)-menthyloxyacetic acid, (-)-diacetyl-L-tartaric acid, (+)-monomethyl-D-tartaric acid, (-)-monomethyl-L-tartaric acid, (-)-diacetone-2-ketogulonic acid, (-)-quinic acid, D-glutamic acid, L-glutamic acid, (S)-(-)-pyrrolidone-5-carboxylic acid, (R)-(-)-2-phenylpropionic acid, (S)-(+)-2-phenylpropionic acid, (S)-1-(2-naphthylsulfonyl)pyrrolidine-2-carboxylic acid, and (S)-1-(4-toluenesulfonyl)pyrrolidine-2-carboxylic acid.

**4.** An uterine smooth muscle relaxing agent comprising a levo-rotatory enantiomer of benzyl alcohol compound represented by the following formula (I):

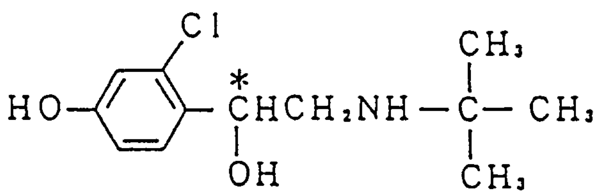

(I)

wherein * C represents an asymmetric carbon atom or a pharmacologically acceptable salt of the compound.

**5.** A bladder smooth muscle relaxing agent comprising a levo-rotatory enantiomer of benzyl alcohol compound represented by the following formula (I):

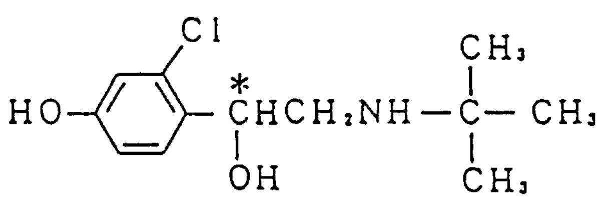

(I)

wherein * C represents an asymmetric carbon atom or a pharmacologically acceptable salt of the compound.

**6.** A pharmaceutical composition for the treatment of premature labor or incontinence of urine comprising an effective amount of a levo-rotatory enantiomer of benzyl alcohol compound represented by the following formula (I):

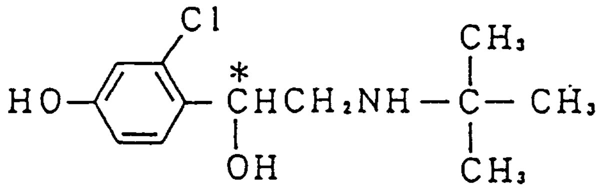

(I)

wherein * C represents an asymmetric carbon atom or a pharmacologically acceptable salt of the compound together with a pharmaceutically acceptable carrier or coating.

**Patentansprüche**

1. Linksdrehendes Enantiomer einer Benzylalkohol-Verbindung, dargestellt durch die folgende Formel (I):

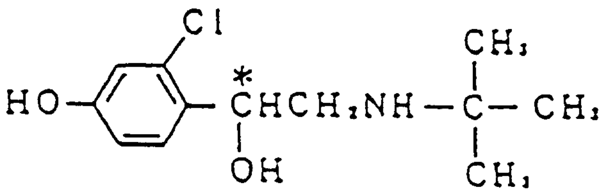

(I)

worin *C ein asymmetrisches Kohlenstoff-Atom bedeutet, sowie pharmazeutische annehmbare Salze der Verbindung.

2. Verfahren zur Herstellung eines linksdrehenden Enantiomers einer Benzylalkohol-Verbindung, dargestellt durch die folgende Formel (I):

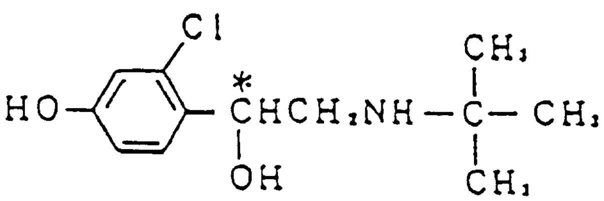

(I)

worin *C ein asymmetrisches Kohlenstoff-Atom bedeutet, sowie pharmazeutisch annehmbarer Salze der Verbindung, umfassend die Schritte der Umsetzung einer racemischen Benzylalkohol-Verbindung, dargestellt durch die folgende Formel (II):

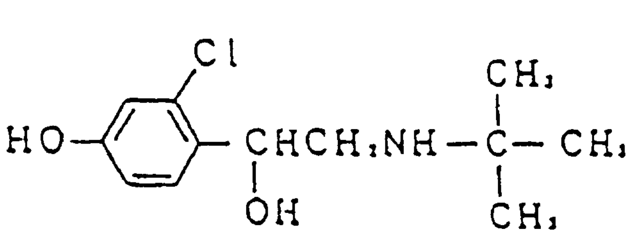

(II)

zu deren Salz unter Verwendung eines Reagens für die Antipodentrennung, fraktionierte Kristallisation des Salzes und nötigenfalls Umsetzung des Salzes zur freien Base.

3. Verfahren zur Herstellung des linksdrehenden Enantiomers nach Anspruch 2, wobei das Reagens für die Antipodentrennung ausgewählt aus der Gruppe bestehend aus D-Campher-10-sulfonsäure, L-Campher-10-sulfonsäure, (+)-Dibenzoyl-D-weinsäure, (-)-Dibenzoyl-L-weinsäure, L-(+)-Weinsäure, D-(-)-Weinsäure, L-(+)-Mandelsäure, D-(-)-Mandelsäure, d-Camphersäure, D-Äpfelsäure, L-Äpfelsäure, (+)-Di-p-toluoyl-D-weinsäure, (-)-Di-toluoyl-L-weinsäure, (-)-Menthyloxyessigsäure, (-)-Diacetyl-L-weinsäure, (+)-Monomethyl-D-weinsäure, (-)-Monomethyl-L-weinsäure, (-)-Diaceton-2-ketogulonsäure, (-)-Chinasäure, D-Glutaminsäure, L-Glutaminsäure, (S)-(-)-Pyrrolidon-5-carbonsäure, (R)-(-)-2-Phenylpropionsäure, (S)-(+)-2-Phenylpropionsäure, (S)-1-(2-Naphthylsulfonyl)pyrrolidin-2-carbonsäure und (S)-1-(4-Toluolsulfonyl)pyrrolidin-2-carbonsäure.

**4.** Relaxans für die glatten Muskeln des Uterus, umfassend ein linksdrehendes Enantiomer einer Benzylalkohol-Verbindung, dargestellt durch die folgende Formel (I):

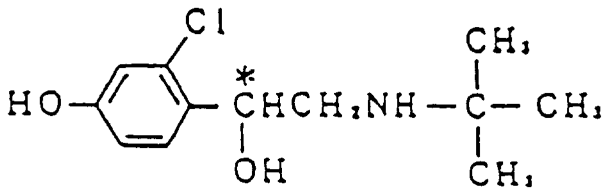

(I)

worin *C ein asymmetrisches Kohlenstoff-Atom bedeutet, oder ein pharmazeutisch annehmbares Salz der Verbindung.

**5.** Relaxans für die glatten Muskeln der Blase, umfassend ein linksdrehendes Enantiomer einer Benzylalkohol-Verbindung, dargestellt durch die folgende Formel (I):

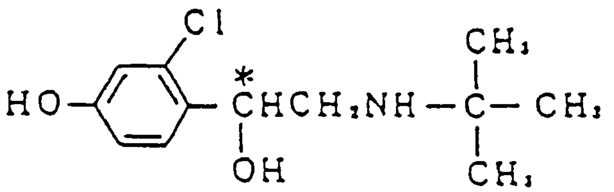

(I)

worin *C ein asymmetrisches Kohlenstoff-Atom bedeutet, oder ein pharmazeutisch annehmbares Salz der Verbindung.

**6.** Pharmazeutische Zusammensetzung für die Behandlung von Frühgeburt oder Harninkontinenz, umfassend eine wirksame Menge eines linksdrehenden Enantiomers einer Benzylalkohol-Verbindung, dargestellt durch die folgende Formel (I):

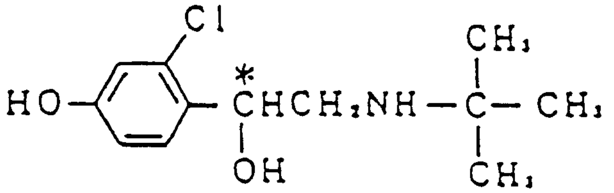

(I)

worin *C ein asymmetrisches Kohlenstoff-Atom bedeutet, oder ein pharmazeutisch annehmbares Salz der Verbindung zusammen mit einem pharmazeutisch annehmbaren Träger oder einer solchen Beschichtung.

**Revendications**

1. Un énantiomère lévogyre d'un composé d'alcool benzylique représenté par la formule (I) suivante :

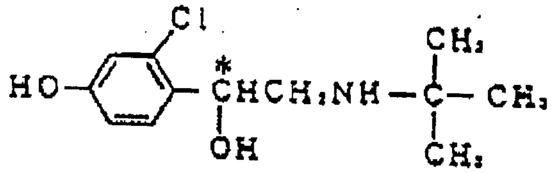

(I)

dans laquelle * C représente un atome de carbone asymétrique ; et les sels pharmacologiquement acceptables du composé.

2. Un procédé de préparation d'un énantiomère lévogyre d'un composé d'alcool benzylique représenté par la formule (I) suivante :

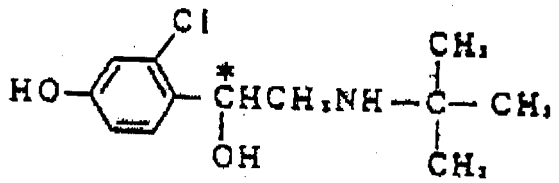

(I)

dans laquelle * C représente un atome de carbone asymétrique, et d'un sel pharmacologiquement acceptable du composé, comprenant les étapes de transformation d'un composé d'alcool benzylique racémique représenté par la formule (II) suivante :

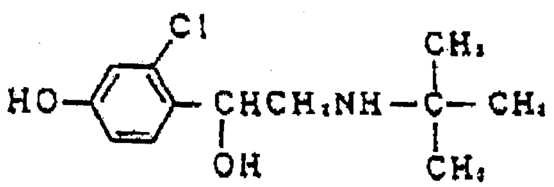

(II)

en un sel de celui-ci en utilisant un réactif pour la résolution optique, une recristallisation fractionnée du sel et, si nécessaire, la transformation du sel en une base libre.

3. Le procédé de préparation de l'énantiomère lévogyre conforme à la revendication 2, le réactif pour la résolution optique est choisi dans le groupe constitué de l'acide D-camphro-10-sulfonique, l'acide L-camphro-10-sulfonique, l'acide (+)-dibenzoyl-D-tartrique, l'acide (-)-dibenzoyl-L-tartrique, l'acide L-(+)-tartrique, l'acide D-(-)-tartrique, l'acide L-(+)-mandélique, l'acide D-(-)-mandélique, l'acide d-camphorique, l'acide D-malique, l'acide L-malique, l'acide (+)-di-p-toluoyl-D-tartrique, l'acide (-)-di-toluoyl-L-tartrique, l'acide (-)-méthyloxyacétique, l'acide (-)-diacétyl-L-tartrique, l'acide (+)-monométhyl-D-tartrique, l'acide (-)-monométhyl-L-tartrique, l'acide (-)-diacétone-2-cétogulonique, l'acide (-)-quinique, l'acide D-glutamique, l'acide L-glutamique, l'acide (S)-(-)-pyrrolidone-5-carboxylique, l'acide (R)-(-)-2-phénylpropionique, l'acide (S)-(+)-2-phénylpropionique, l'acide (S)-1-(2-naphtylsulfonyl)pyrrolidine-2-carboxylique et l'acide (S)-1-(4-toluènesulfonyl)pyrrolidine-2-carboxylique.

4. Un agent de décontraction du muscle lisse utérin comprenant un énantiomère lévogyre d'un composé d'alcool benzylique représenté par la formule (I) suivante :

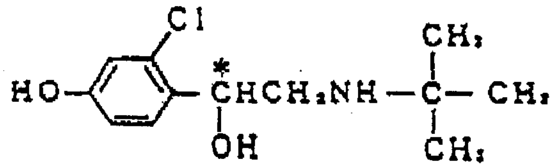

(I)

dans laquelle * C représente un atome de carbone asymétrique, ou un sel pharmacologiquement acceptable du composé.

5. Un agent de décontraction du muscle lisse de la vessie comprenant un énantiomère lévogyre d'un composé d'alcool benzylique représenté par la formule (I) suivante :

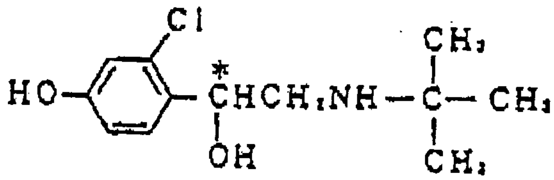

(I)

dans laquelle * C représente un atome de carbone asymétrique, ou un sel pharmacologiquement acceptable du composé.

6. Une composition pharmaceutique pour le traitement du travail prématuré ou de l'incontinence urinaire comprenant une quantité efficace d'un énantiomère lévogyre d'un composé d'alcool benzylique représenté par la formule (I) suivante :

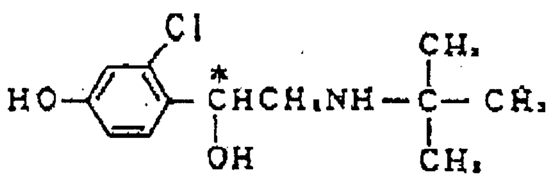

(I)

dans laquelle * C représente un atome de carbone asymétrique, ou un sel pharmacologiquement acceptable du composé, en combinaison avec un support ou une enveloppe pharmaceutiquement acceptable.